# EUROPEAN PATENT APPLICATION

(11) **EP 2 465 515 A1**
(43) Date of publication of application: **20.06.2012**
(21) Application number: 11193982.3
(22) Date of filing: 16.12.2011
(51) Int. Cl.: A61K 35/74, C12N 1/20

(54) **Cyanobacterium of the family pseudanabaenaceae (oscillatoriales, cyanophyta), genus protolyngbya, species protolyngbya sp. Strain ITD-01**

(30) Priority: 16.12.2010 IT PD20100380
(71) Applicant: Associazione Termalisti Isola d'Ischia, 80077 Ischia Porto (IT)
(72) Inventor: Andreoli, Carlo, 35020 Albignasego (IT); Rascio, Nicoletta, 35020 Albignasego (IT); Moro, Isabella, 35136 Padova (IT); Sciuto, Katia, 32100 Belluno (IT)
(74) Representative: Gervasi, Gemma

(57) **Abstract**

It is herein described the identification and characterization of a new strain of cyanobacterium belonging to the family Pseudanabaenaceae (Oscillatoriales, Cyanophyta), genus *Protolyngbya,* designated strain ITD-01, found on the surface of the Ischia thermal muds. This cyanobacterium is found to be particularly rich in C-phycocyanin.

SEQUENCE LISTING

<110>  Associazione Termalisti Isola di Ischia

<120>  CYANOBACTERIUM OF THE FAMILY PSEUDANABAENACEAE (OSCILLATORIALES,
       CYANOPHYTA), GENUS PROTOLYNGBYA, SPECIES PROTOLYNGBYA SP. STRAIN
       ITD-01

<130>  10780PTEP

<150>  PD2010A000380
<151>  2010-12-16

<160>  2

<170>  PatentIn version 3.5

<210>  1
<211>  979
<212>  DNA
<213>  Protolyngbya sp. strain ITD-01


<220>
<221>  rDNA
<222>  (1)..(979)
<223>  gene 16S rDNA

<400>  1
ggggtccttc gggacttagt ggcggacggg tgagtaacgc gtgaggatct ggcttcagga      60

cggggataac attgggaaac tgatgctaat acccgatatg gcgagagctg aaagattaat     120

tgcctgaaga tgaactcgcg tccgattagc tagttggagt ggtaaaggcm cccccarggc     180

gatgatcggt agctggtctg agaggatgat cagccacact gggactgaga cacggcccag     240

actcctacgg gaggcagcag tggggaattt tccgcaatgg gcgaaagcct gacggagcaa     300

taccgcgtgc gggaggacgg cctttgggtt gtaaaccgct tttctcaggg aagaagatct     360

gacggtacct gaggaatcag catcggctaa ctccgtgcca gcagccgcgg taatacggag     420

gatgcaagcg ttatccggaa ttattgggcg taaagcgtcc gcaggtggtt ggtcaagtct     480

gctgtcaaag cgtggagctt aactccataa gggcggtgga aactgattaa ctagaggatg     540

gtaggggtca cgggaattcc cagtgtagcg gtgaaatgcg tagatattgg gaagaacacc     600

agcggcgaaa gcgcgtgact gggccattac tgacactcat ggacgaaagc taggggagcg     660

aaagggatta gatacccctg tagtcctagc tgtaaacgat ggacactagg tgttgcccgt     720

atcgacccgg gcagtgccgt agccaacgcg ttaagtgtcc cgcctgggga gtacgcacgc     780

aagtgtgaaa ctcaaaggaa ttgacggggg cccgcacaag cggtggagta tgtggtttaa     840

ttcgatgcaa cgcgaagaac cttaccaggg tttgacatcc ttcgaatcct tgggaaaccg     900

aggagtgcct tcgggagcga agagacaggt ggtgcatggc ygtcgtcagc tcgtgtcgyg     960

agatgttggg ttaagtccc                                                  979

<211>  1183
<212>  DNA
<213>  Protolyngbya sp. ceppo ITD-01

<220>
<221>  gene
<222>  (1)..(1183)
<223>  gene rbcL DNA

<400>  2
tgacctcgat cgctacaagg gacgctgcta cgatatcgag ccagttcccg gcgaagataa      60

ccaatttatc gcctacattg cctatcctct cgatctattc gaggaagggt ctgtaaccaa     120

catgctgacc tctatcgtcg gtaacgtgtt tggtttcaaa gctctgaaag ctctgcgtct     180

ggaagacctg cggattccgg ttgcttacct gaagaccttc caaggccctc cccacggtat     240

ccaagttgag cgcgacaaga ttaataagta tggtcgtcct ctgttgggtt gcaccatcaa     300

gcctaagctc ggtctgtctg ctaagaacta tggccgtgcc gtttatgagt gtctgcgggg     360

tggtctagac ttcaccaaag acgacgaaaa catcaactcg cagccgttcc aacgctggcg     420

cgatcgcttc ctatttgtgg ctgatgcaat ccataagtct caagcagaaa cgggcgaaat     480

caagggccac tacctcaacg tgactgctgc aacctgcgaa gacatgctga agcgggctga     540

gtttgcgaaa gaactcggaa tgcccatcat catgcatgac tttttgacag ctggcttcac     600

tgccaacaca actctgtcga agtggtgtcg cgacaacggt gtgctgctgc acattcaccg     660

tgcaatgcac gcagtgatcg accgtcaaaa gaaccacggg atgcacttcc gcgttctggc     720

gaagtgcttg cggatgtcgg gtggagacca cattcacacc ggaactgtcg tcggtaagct     780

tgagggtgac aaagccgtaa ccctcggttt cgtagacctc cttcgcgaaa actacgttga     840

gcaagaccgc tcgaagggta tctacttcac ccaagactgg gcttccatgc ctggtgtaat     900

ggctgtggct tctggtggta tccacgtatg gcacatgcct gctctggtag aaatctttgg     960

cgacgactct gttctccagt ttggtggtgg tactctgggt cacccctggg gtaatgcgcc    1020

gggcgcaact gctaaccggg ttgctctgga agcttgcgtt caagctcgta acgaaggtcg    1080

tgacctgatg cgtgaaggtg gcgacatcat tcgtgaagct tgccgctggt cacctgagtt    1140

ggcaactgct tgcgaactgt ggaaagaaat caagttcgag tta                      1183

## Description

### Field of the invention

The invention relates to a new strain of cyanobacterium of the family Pseudanabaenaceae (Oscillatoriales, Cyanophyta), identified as *Protolyngbya* sp. strain ITD-01, found in the thermal muds of the Ischia basin and usefully employable in the pharmaceutical and cosmeceutical field and for foodstuffs, being characterized by a high content of C-phycocyanin.

### Prior art

The Ischia Thermal District has been known since antiquity for the curative effects of the thermal waters in numerous pathologies. The thermal waters of the island of Ischia are in fact particularly indicated in chronic rheumatic diseases, after-effects of fractures, chronic diseases of the bronchi, of the ear, nose and throat. From the XVII^{th} up to the middle of the XX^{th} century, a number of establishments were founded close to the most important thermal springs, which now constitute the Thermal District of the Island of Ischia. Information regarding the Thermal District of Ischia is limited to the history and geology of the island, but little is known about the biodiversity of this environment. The thermal-mineral waters of Ischia, because of the varying chemical composition of the ground of the territory where they flow, can be divided into bicarbonate-calcic, bicarbonate-alkaline, sulphate-chloride-alkaline waters and transitional waters. Moreover, the temperature of the water at the various establishments can vary from 15°C to 86°C. Due to the notable variability of the physicochemical parameters of the waters, various organisms can be found at the different sites of the Ischia District, including bacteria, protozoa, microalgae and cyanobacteria. The latter are known for their ability to produce various bioactive compounds that can be utilized in several applications, including medical treatments (Singh, S. et al., 2005, Bioactive Compounds from Cyanobacteria and Microalgae: An Overview, Crit. Rev. Biotechnol. 25: 73-95; Eriksen N.T., 2008, Production of phycocyanin - a pigment with applications in biology, biotechnology, foods and medicine, Appl. Microbiol. Biotechnol. 1: 1-14; Gerwick, W.H. et al., 2008, Giant Marine Cyanobacteria produce exciting potential pharmaceuticals, Microbe 3: 277-284; Sivonen, K. & Börner, T., 2008, Bioactive compounds produced by cyanobacteria, In: Herraro A. & Flores E. (eds): The cyanobacteria: Molecular Biology, Genomics and Evolution: 159-197, Caister Academic Press, Norfolk).

It was demonstrated recently that the therapeutic effectiveness of mud therapy is not linked simply to the beneficial effects of the heat and electrolytes, but is due to the presence of substances with high anti-inflammatory activity synthesized by the microorganisms that colonize the thermal mud during the process of maturation. The muds used in the thermal treatments are colonized by a complex community of microorganisms, the most representative of which, in terms of extent of colonization and in biomass produced, are found to be certain species of cyanobacteria. These microorganisms are able to produce a whole range of secondary metabolites (polyunsaturated fatty acids, beta-carotene, lycopene, hexopolysaccharides, C-phycocyanin, etc.) which form the basis of the characteristic therapeutic powers of the thermal muds. Among these compounds, C-phycocyanin is found to be particularly important and can be used both in the therapeutic field and the cosmeceutical field.

Regarding C-phycocyanin, as well known, this is the most abundant pigment, and can reach approx. 15% of the dry weight in cyanobacterial cells. The chemical structure of the chromophores in phycocyanin (linear tetrapyrroles) is very similar to that of bilirubin and it is a physiological antioxidant that removes peroxide radicals. It has, in fact, been demonstrated that both *in vitro* and *in vivo,* C-phycocyanin acts as an antioxidant, removing oxygen free radicals (Romay, C. et al., 1998, Inflammation Res., 47: 36-41; Bhat V.B. et al.; 2001, Scavenging of peroxynitrite by phycocyanin and phycocyanobilin from Spirulina platensis: protection against oxidative damage to DNA, Biochem. Bioph. Res. Comm. 285: 262-266).

Among the various beneficial effects of C-phycocyanin, we should also mention that of preventing liver damage due to a poorly balanced diet. In this connection, it has been demonstrated that phycocyanin reduces the free radicals produced by administration of CCl₄, acting as a hepatoprotective agent (Vadiraja, B.B. et al., 1998, Hepatoprotective effect of C-phycocyanin: Protection for carbon tetrachloride and R-(+)-pulegone-mediated hepatotoxicity in rats, Biochem. Biophys, Res. Comm., 249: 428-431).

Furthermore, C-phycocyanin is used in the food industry (Yoshida, A. et al., 1996, Enzyme immunoassay for phycocyanin as the main component of Spirulina color in foods, Biosci. Biotechnol. Biochem. 60: 57-60) and in cosmetics (Cohen Z., 1986, Products from microalgae, In: Richmond, A. (eds.), Handbook of Microalgal Mass Culture, CRC Press, Boca Raton, pp. 421-454).

### Summary

The invention relates to a new strain of cyanobacterium identified as ITD-01 (ITD = Ischia Thermal District), belonging to the order Oscillatoriales and to the family Pseudanabaenaceae (subfamily: Leptolyngbyoideae; genus: *Protolyngbya;* species: *Protolyngbya* sp.), deposited under No. CCALA 945 on 4 November 2010 in the Culture Collection of Autotrophic Organisms (CCALA), Institute of Botany, Academy of Sciences of the Czech Republic, Centre of Phycology, Dukelská 135, TREBON CZ-379 82. The cyanobacterium *Protolyngbya* sp. strain ITD-01 is **characterized in that** it comprises the rDNA sequence ID NO: 1 for the 16S rDNA gene and the DNA sequence ID NO: 2 for the *rbc*L plastid gene.

The invention further relates to the strain derivatives thereof obtained by genetic mutation or transformation or by growing in selective conditions. Products obtained by extraction, subcellular fractionation and/or lysis of cultures containing the bacterium and its bioactive compounds are also regarded as derivatives of the cyanobacterium *Protolyngbya* sp. strain ITD-01. Among the active principles present in the ITD-01 strain, C-phycocyanin constitutes the predominant water-soluble pigment, reaching high percentages (68%). These bioactive compounds are obtained by extraction from bulk cell cultures of cyanobacteria.

According to a main aspect of the invention, the cyanobacterium ITD-01 and the derivatives thereof, as previously defined, constitute the active principles that can be used in the pharmaceutical and food industry, as well as in cosmetics. In particular, the main advantage for the use of the cyanobacterium ITD-01 in the field of cosmetics, pharmaceuticals and dietary supplements can be attributed to the notable amount of C-phycocyanin. Moreover, the presence of the ITD-01 strain, exclusively from the thermal environment of the island of Ischia, in thermal muds makes these muds unique.

Therefore, according to a further aspect, the invention relates to a use of the microorganism according to the invention or of the derivatives thereof for preparing compositions in the field of pharmaceuticals, cosmetics and/or food supplements.

### Brief description of the drawings

Figs. 1-3. The figures show several images of the strain ITD-01 found on the surface of the thermal mud of Ischia:
   Fig. 1. Light-microscope image of long filaments, very thin, unbranched. Scale bar = 5 µm.
   Fig. 2. Scanning electron microscope image of trichomes characterized by cylindrical cells and by distinct constrictions on the transverse walls (arrow). Scale bar = 1 µm.
   Fig. 3. Transmission electron microscope image of a trichome characterized by cells with parallel thylakoids (t), located at the periphery of the cells, and surrounded by a mucilaginous sheath (s). Scale bar = 1 µm.
   Fig. 4. In the figure are reported the percentages of the various phycobiliproteins present in the cyanobacterium ITD-01: PC= phycocyanin; APC= allophycocyanin; PE= phycoerythrin.
   Fig. 5. The figure shows the phylogenetic tree based on the sequences of the 16S rRNA gene and constructed using the *maximum-likelihood* method. The GenBank accession numbers are given in parentheses. The scale bar represents 0.02 nucleotide substitutions per site.
   Fig. 6. The figure shows the phylogenetic tree based on the sequences of the *rbc*L gene and constructed using the *maximum-likelihood* method. The GenBank accession numbers are given in parentheses. The scale bar represents 0.05 nucleotide substitutions per site.

### Detailed description of the invention

1. The microorganism ITD-01 that is the object of the present patent was isolated from a blue-green felt forming on the surface of the thermal muds of the hotel NH Ischia Thermal Spa Resort (Naples, Italy) (40°44'0" North, 13°36'0" East) in April 2009. The temperature of the water at the time of sampling was about 30°C and the pH was 7. The organism isolated was put in a liquid culture medium BG11 (Rippka et al. 1979, Generic Assignments, Strain Histories and Properties of Pure Cultures of Cyanobacteria, J. Gen. Microbiol. 111: 1-61) at a growing temperature of 30°C and at a luminous intensity of 35 µmol photons m⁻² s⁻¹ with a photoperiod of 12/12 hours.

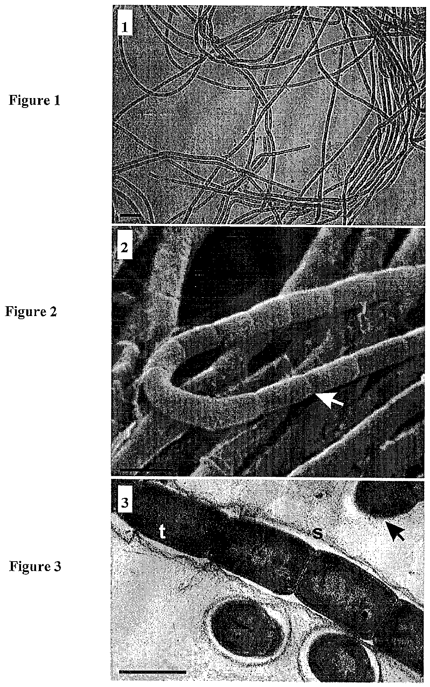

The strain ITD-01 was characterized using a multi-step type approach, comprising morphological, ultrastructural (SEM and TEM), biochemical and molecular characterizations. The morphological analyses show that the strain ITD-01 consists of long filaments of blue-green colour, very thin, unbranched (Fig. 1). The filaments are isopolar, very close together so that they form compact felts on the substrate. Each filament, called a trichome, is composed of cells that are longer than wide, with dimensions varying from 0.8 to 1.5 µm in length and from 0.7 to 0.9 µm in width (Fig. 2). The filaments show distinct constrictions on the transverse walls (Fig. 2). In the transmission electron microscopy, the cells are characterized by the presence of 3-4 thylakoid membranes, arranged parallel to the periphery of the cell, and some inclusions. In addition, each filament is surrounded by a colourless sheath (Fig. 3). No gas vesicles are present. The cells reproduce by binary division.

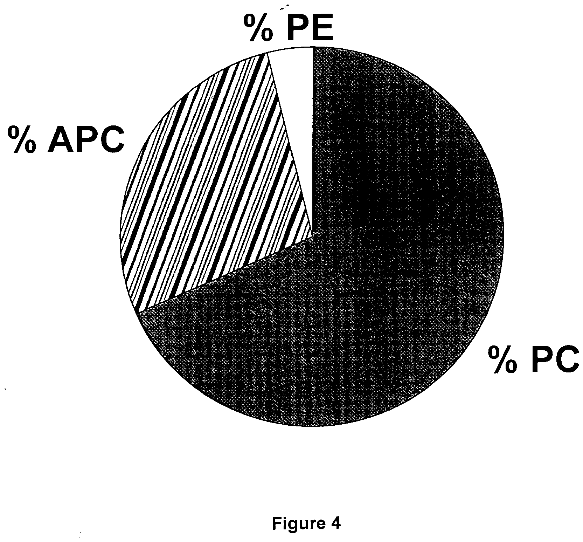

Spectrophotometric analyses, performed for determining the composition of the various phycobiliproteins, show the presence of C-phycocyanin (68%), allophycocyanin (28%) and phycoerythrin (4%) (Fig. 4). The high proportion of C-phycocyanin (68%), a highly fluorescent water-soluble protein, present in the cyanobacteria and used in the food industry (Yoshida et al., 1996, *ref.cít*.), in cosmetics (Cohen, 1986, *ref. cit*.), but known in particular for its pharmacological properties (Romay et al. 1998, *ref. cit*.;. Vadiraja et al. 1998, *ref. cit*.;. Bhat et al. 2001, *ref. cit*.), suggests how this organism might contribute to the therapeutic properties of the Ischia mud.

Analyses performed using HPLC show the presence of the following fat-soluble pigments: myxol-2'-glycoside, nostoxanthin, caloxanthin, zeaxanthin, chlorophyll a, trans-β- and cis-β-carotene.

In addition to morphological, ultrastructural and biochemical characterization, molecular analyses were carried out for more precise taxonomic identification of the strain of cyanobacterium under consideration. Genetic characterization was performed according to an approach that envisages the use of different molecular markers. For this purpose, the 16S rDNA gene (SEQ ID NO: 1) and the plastid gene *rbc*L that codes for the large subunit of the enzyme ribulose-1,5-bisphosphate carboxylase/oxygenase (SEQ ID NO: 2) were used as markers. Alignment of the sequence of the 16S rDNA gene of the strain ITD-01 with sequences of the same gene available in GenBank identifies a percentage identity of 94% between ITD-01 and a strain of *Leptolyngbya,* strain FYG (GenBank FJ933259), isolated from felts present in hot springs of Yellowstone National Park (USA).

For its part, the DNA sequence of the *rbc*L gene of ITD-01 has a percentage identity of 85% with the corresponding sequences of *Leptolyngbya laminosa* ETS-08 (a strain isolated from the Euganean Thermal District) and of *Leptolyngbya* sp. PCC 73110 and of 84% with *Pseudanabaena persicina* CCMP638.

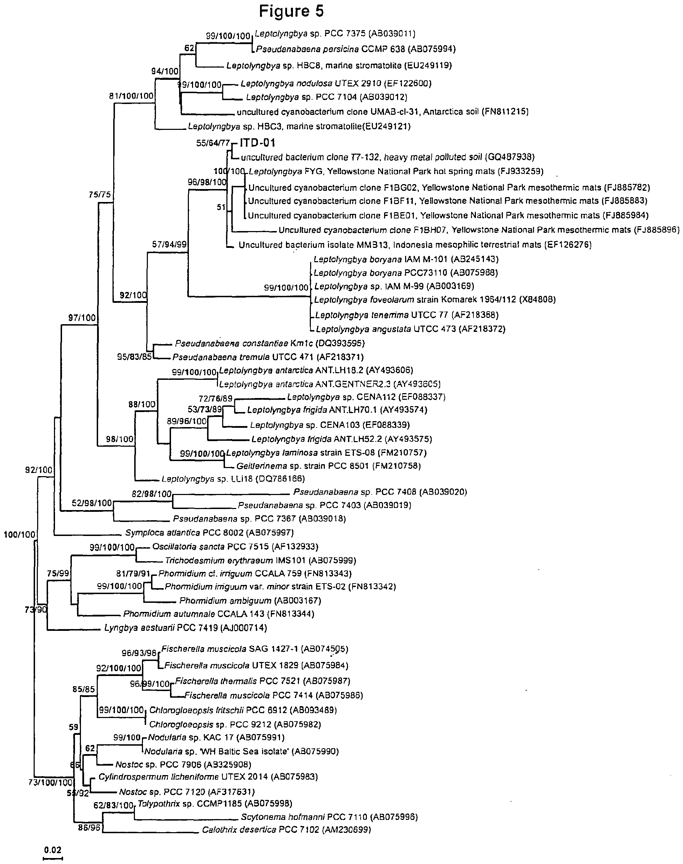

The topology obtained with the sequences of the 16S rDNA gene (Fig. 5) shows that the Ischia strain ITD-01 belongs to the order Oscillatoriales, family Pseudanabaenaceae, and to the actual genus *Leptolyngbya.* Based on its position in the topology obtained with the 16S rDNA gene, its morphology corresponding to the description given for the subgenus *Protolyngbya* and in the absence of other sequences available in the public databases for the members of this subgenus, it is thought that ITD-01 can be the first strain sequenced of the subgenus *Protolyngbya.* Moreover, the phylogenetic analyses, as well as the morphological characteristics, lead us to propose establishment of the new genus *Protolyngbya,* a separate genus from the genus *Leptolyngbya.*

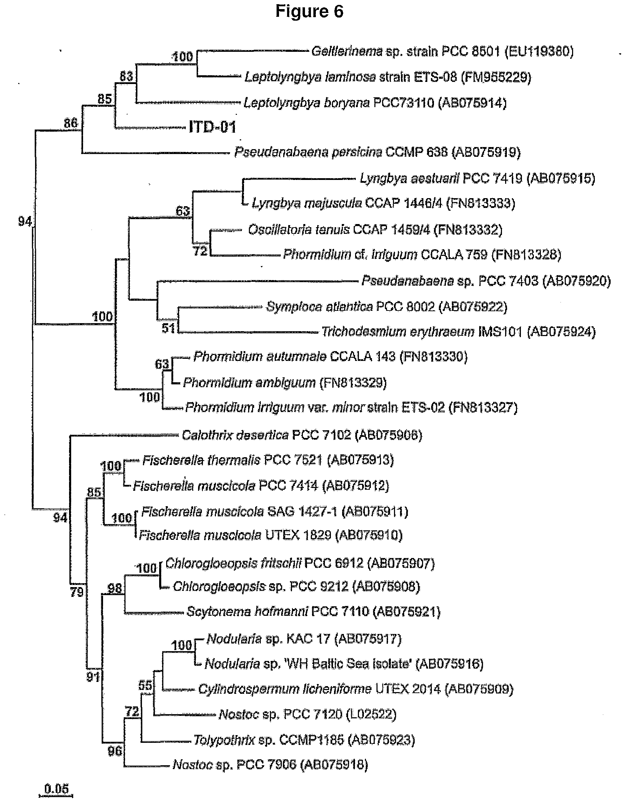

Furthermore, the phylogenetic tree obtained with the sequences of the *rbc*L gene (Fig. 6), although considering a smaller number of organisms owing to the small number of sequences available in GenBank for this gene, provides evidence that the strain ITD-01 belongs to the family Pseudanabaenaceae. Also in this topology, the strain in question is found to be a 'sister taxon' of a group consisting of some forms belonging to the subfamily Leptolyngbyoideae.

In light of the investigations that have been carried out, the results obtained suggest that the strain ITD-01 might represent the first taxon described belonging to a new genus, the name of which could be *Protolyngbya.*

The strain ITD-01 No. CCALA 945 and the strain derivatives thereof can be maintained in liquid or solid culture on a suitable culture medium, or stored in lyophilized, or frozen form. Methods of storage, maintenance and growth of bacteria are known in the literature and are described for example in: MacFaddin, J. F. 1985, Media for isolation, cultivation, identification, maintenance of medical bacteria. Williams & Wilkins, Baltimore, Md., or in Ted R. Johnson, Christine L. Case, James G. Cappuccino, Natalie Sherman, Virginia Schurman, Janet Savage, 1999. Biology 201: Microbiology Laboratory Manual. Addison-Wesley edition. Some procedures for carrying out the invention are illustrated below, constituting only some examples thereof and therefore are neither exhaustive, nor limiting of what was disclosed above. Furthermore, the analyses are described that led to the characterization of strain ITD-01 No. CCALA 945 isolated from the muds of the thermal district of Ischia.

### EXPERIMENTAL PART

### Isolation and maintenance in culture of the cyanobacterium of the family Pseudanabaenaceae (Oscillatoriales, Cyanophyta) strain ITD-01

The microorganism ITD-01 was isolated from a blue-green felt forming on the surface of the thermal muds of the hotel NH Ischia Thermal Spa Resort (Naples, Italy) (40°44'0" North, 13°36'0" East) in April 2009. The temperature of the water at the time of sampling was about 30°C and the pH was 7.

After collecting the felt, the organism was isolated in the laboratory by the technique of plating on agar. This technique consists of inoculating a pool of microorganisms in a Petri dish, containing a culture medium solidified by adding agar, a complex polysaccharide that is obtained from red algae. In our case an agar-containing BG11 culture medium was used. The purpose of this technique is to separate the various forms of microorganisms forming the population. The cells of the various organisms can reproduce in the solid culture medium and form various clusters called colonies. One of these colonies, constituted solely of cells of the organism under investigation, was then inoculated in a liquid culture medium BG11 (Rippka et al. 1979, *ref. cit*.) and was left to grow at a temperature of 30°C and a luminous intensity of 35 µmol photons m⁻² s⁻¹ with a photoperiod of 12/12 hours.

Having obtained bulk cultures of the organism ITD-01, the various investigations were carried out for determining its exact systematic classification.

### Morphological and ultrastructural investigations

The observations with the light microscope were performed using a Leica DM5000 light microscope, working both in visible light and in fluorescence and equipped with a digital image analyser. For the observations with the light microscope in fluorescence, the samples were excited with UV light (340-380 nm) which permits visualization of the characteristic red autofluorescence of chlorophyll.

For the observations with the scanning electron microscope (SEM), sample aliquots were taken from the liquid culture. These were fixed for 24 h in 6% glutaraldehyde in cacodylate buffer 0.2 M pH 6.9, then post-fixed overnight in 1% osmium tetroxide in cacodylate buffer 0.2 M pH 6.9 and then dehydrated in aqueous solutions of ethanol at increasing concentrations.

Next, the samples were submitted to "*critical point drying*", mounted on suitable supports, metallized with gold and observed with a Stereoscan 260-Cambridge scanning electron microscope.

For the ultrastructural analyses, conducted with the transmission electron microscope (TEM), samples were taken from the liquid culture. These were fixed in glutaraldehyde at 6%, in cacodylate buffer 0.2 M pH 6.9 overnight at 4°C. The next day, after 3 washings of 10 minutes each with cacodylate buffer, they were post-fixed in 1% osmium tetroxide in cacodylate buffer 0.2 M pH 6.9, followed by dehydration in aqueous solutions of ethanol at increasing concentrations. They were embedded in Araldite. The samples embedded in resin were then cut using an ultramicrotome (Ultracut Reichert-Jung) to obtain ultrathin sections with thickness of 80-90 nm. These were collected on copper screens covered with a film of collodion and with a thin layer of carbon.

The screens were then contrasted with lead citrate for 10 minutes in the dark, washed thoroughly by washings with distilled water and then dried.

The sections were observed with a Hitachi H300 transmission electron microscope operating at 75 kV.

Figs. 1-3 show examples of some images obtained with the light microscope (Fig. 1) and electron microscope (Figs. 2 and 3), as already mentioned.

### Biochemical analyses

Samples were taken from the culture. The samples were weighed to obtain an estimate of the fresh weight and were then put in a test tube with quartz sand and pounded with a pestle until a complete trituration . For extraction of the phycobiliproteins, 1 ml of phosphate buffer (NaH₂PO₄ 0.01M, NaCl 0.15M, pH 7) was added to the triturated sample. The samples were left at 4°C overnight and then centrifuged at 14000 *g* for 2 min. The aqueous phase was removed and then analysed with a spectrophotometer (Beckman DU-530). The absorbance of the samples was detected at wavelengths of 562 nm for phycoerythrin (PE), 620 nm for phycocyanin (PC), 652 nm for allophycocyanin (APC). The concentrations of the phycobiliproteins were calculated using suitable formulae.

The fat-soluble photosynthetic pigments (chlorophyll a and carotenoids) were extracted and then analysed by high-performance liquid chromatography (HPLC).

For this analysis, samples of ITD-01, collected from the cyanobacterial cultures, were put in test tubes, to which 500 µ1 of 90% acetone and a suitable amount of quartz sand were added. The whole was pounded with a pestle and then centrifuged at 14000 *g* for 2 minutes. The supernatant was collected and dried with gaseous nitrogen and then 100 µl of 100% acetone was added. At this point the sample was analysed by HPLC using the method described by Komárek, J. et al. (Komárek, J. et al., 1999, Generic characters of the simplest cyanoprokaryotes Cyanobium, Cyanobacterium and Synechococcus, Cryptogamie Algol. 20: 209-222).

Fig. 4 shows the results obtained, which have already been mentioned.

### Molecular analyses

The genomic DNA was extracted and purified using the *Genomic DNA purification kit* (FERMENTAS).

The concentration of DNA was determined by a spectrophotometric analysis, performed with a Beckman DU-530 spectrophotometer at wavelengths of 230, 260, 280 and 320 nm, which also made it possible to evaluate the degree of purity and protein contamination of the DNA.

The first molecular marker tested was the 16S rDNA gene (SEQ ID NO: 1). The primers selected for amplification of the genomic fragment corresponding to 16S rDNA, designated 16S1 (5'-AGAGTTTGATCCTGGCTCAG-3') (position +1-+20, GenBank accession number D83715) and 16S2 (5'-ACGGCTACCTTGTTACGACTT-3') (position +1453-+1433, GenBank accession number D83715), were developed by Kobayashi (unpublished data) on the genetic sequence of 16S rDNA of *Synechococcus elongatus* (GenBank accession number D83715). For sequencing, however, primers designed on the alignment of the genetic sequences of 16S rDNA of the following cyanobacteria were used: *Phormidium ambiguum* AB003167; *Stanieria cyanosphaera* AF132931; *Anabaena cylindrica* AF091150; *Nodularia* sp. PCC 9350 AY038034; *Trichodesmium erytraeum* AF013030; *Oscillatoria sancta* AF132933 and *Synechococcus elongatus* D83715, present in the GenBank database of the NCBI *(National Center for Biotechnology Information)* (www.ncbi.nlm.nih.gov/).

The sequences of the primers used for sequencing are as follows.: 16S3: 5'-CTACGGGAGGCAGCAGTG-3' (position +304-+321, GenBank accession number AJ548503); 16S4: 5'-CACTGCTGCCTCCCGTAG-3' (position +321-+304, GenBank accession number AJ548503); 16S5: 5'-ATACCCCAGTAGTCCTAG-3' (position +731-+748, GenBank accession number AJ548503); 16S6: 5'-TAAACCACATACTCCACC-3' (position +898-+881, GenBank accession number AJ548503).

Another marker selected for molecular characterization was the plastid gene *rbc*L (SEQ ID NO: 2). Both for amplification and for sequencing of the locus *rbc*L*,* two pairs of primers were used (GF-AB 5'-CTACGGGAGGCAGCAGTG-3'; GR-D 5'-TGCCAIACGTGGATACCACC-3'; GF-C 5'-CTTYACYCAAGACTGGGCTTC-3'; GR-E 5'-AACTCRAACTTGATTTCYTTCC-3').

The PCR amplification products, with the conditions given in Table 1, were purified with exonuclease I and alkaline phosphatase (ExoSAP-ITTM kit, Amersham Biosciences).

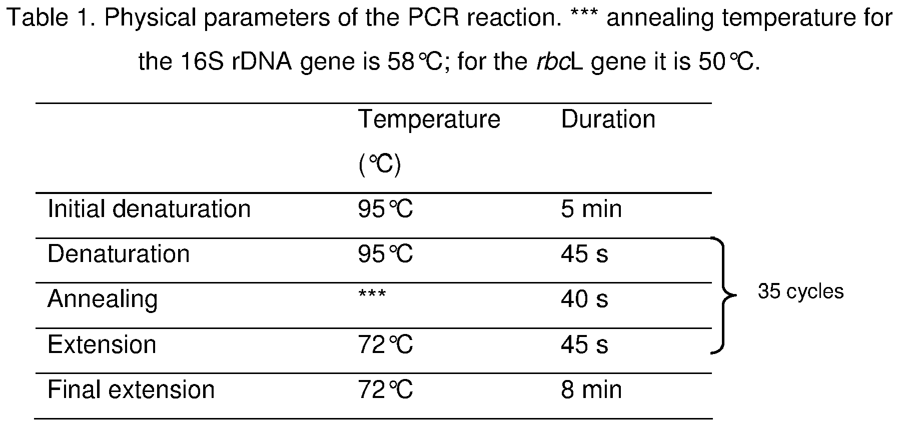

The sequences were determined at BMR Genomics (www.bmr-genomics.it), using the kit ABI PRISM Dye Terminator Sequencing Core (Perkin Elmer). Electrophoresis of the sequence reactions was performed with the automatic sequencer ABI PRISM 377, version 2.1.1.

The nucleotide sequences thus obtained were compared with others using BLAST software, which makes it possible to align and compare sequences of interest with sequences available in the GenBank database. The result of the analysis is an alignment in which a score of identity with the sequence under investigation is assigned to every sequence found in the database.

Using the sequences obtained from the sequencing and the other sequences available in the GenBank database, separate datasets were constructed for the molecular markers selected.

The sequences of each dataset were aligned using the ClustalW software.

On the basis of the datasets constructed, using the *PHYML* 2.4 software, phylogenetic reconstructions were performed according to the *maximum likelihood* (ML) method, applying the model GTR+I+G.

The robustness of each of the topologies obtained was evaluated applying the *Bootstrap* test (BT), with 1000 repetitions.

The phylogenetic trees thus generated were visualized with the *NJplot* software (Figs. 5 and 6).

## Claims

1. *Protolyngbya* sp. ITD-01 cianobacterial strain deposited with N ° CCALA 945 in CCALA, Institute of Botany, Academy of Sciences of the Czech Republic, Centre of Phycology, TREBOIV CZ-379 82 comprising for gene 16S the rDNA SEQ ID NO: 1 and for gene *rbc*L the DNA SEQ ID NO: 2.

2. Strain derivatives of *Protolyngbya* sp. ITD-01 according to claim 1, wherein said strains are obtained by mutation or genetic transformation or by growth under selective conditions thereof.

3. Derivatives *of Protolyngbya* sp. ITD-01 according to claim 1, wherein said derivatives are obtained by extraction, sub-cellular fractionation and/or lysis from cell cultures growth under controlled conditions thereof.

4. Derivatives of *Protolyngbya* sp. ITD-01 according to claim 3 comprising the hydrosoluble C-ficocianin pigment.

5. Compositions comprising as active principle the *Protolyngbya* sp. ITD-01 cianobacterial strain according to claim 1 or derivatives thereof according to claims 2-4.

6. Compositions according to claim 5, wherein the active principle is in combination with one or more other active principles and/or excipients, diluents.

7. *Protolyngbya* sp. ITD-01 cianobacterial strain according to claim 1 or derivatives thereof according to claims 2-4 for use in cosmetics.

8. *Protolyngbya* sp. ITD-01 cianobacterial strain according to claim 1 or derivatives thereof according to claims 2-4 for use in pharmaceutical field.

9. *Protolyngbya* sp. ITD-01 cianobacterial strain according to claim 1 or derivatives thereof according to claims 2-4 for use in the preparation of dietary supplements.

10. *Protolyngbya* sp. ITD-01 cianobacterial strain according to claim 1 or derivatives thereof according to claims 2-4 for use in mud therapy.
